# EUROPEAN PATENT APPLICATION

(11) **EP 0 756 173 A1**
(43) Date of publication of application: **29.01.1997**
(21) Application number: 96901979.3
(22) Date of filing: 09.02.1996
(51) Int. Cl.: G01N 33/53, G01N 33/564

(54) **REAGENT FOR DETECTING SUBSTANCES AND METHOD FOR DIAGNOSING RHEUMATOID ARTHRITIS**

(30) Priority: 10.02.1995 JP 23091/95
(71) Applicant: MORINAGA MILK INDUSTRY CO., LTD., Minato-ku, Tokyo-to 108 (JP); Eisai Co., Ltd., Tokyo 112 (JP)
(72) Inventor: SAKURAOKA, Atsushi, Morinaga Milk Industry Co. Ltd, 5 chome Zama-shi Kanagawa 228 a 228 (JP); AONUMA, Ikue, Morinaga Milk Industry Co., Ltd.,, 5-chome Zama-shi Kanagawa 228 (JP); HARADA, Yoshitsugu, Morinaga Milk Industry Co. Ltd, 5-chome Zama-shi Kanagawa 228 228 (JP); YAMADA, Yuji, Ibaraki 305 (JP)
(74) Representative: Bannerman, David Gardner
(86) International application number: JP9600288
(87) International publication number: WO9624845

(57) **Abstract**

Diagnosis of rheumatoid arthritis or the like is performed accurately, conveniently, and quickly in accordance with the steps of:
reacting a membrane on which one of galactose-deficient IgG and Ricinus communis agglutinin I is immobilized, colored fine particles on which the other is immobilized, and a biological sample collected from a test subject with each other; and
detecting the colored fine particles captured on the membrane through binding between an anti-galactose-deficient IgG antibody contained in the sample and the galactose-deficient IgG and binding between Ricinus communis agglutinin I and galactose contained in the anti-galactose-deficient IgG antibody.

## Description

### Technical Field

The present invention relates to a detecting reagent for detecting a specified substance to be detected, especially anti-galactose-deficient IgG antibodies (antibodies against IgG which is deficient in galactose) found in patients with rheumatoid arthritis. The present invention also relates to a diagnostic agent for rheumatoid arthritis, and a method for detecting rheumatoid arthritis.

### Background Art

Analysis of substances existing in vivo, which is used as a means for performing differential diagnosis of diseases, has been widely adopted in clinical examination. In the clinical examination, a quantitative method having high sensitivity and accuracy and a qualitative method executed by simple operation to obtain a result in a short period of time are appropriately chosen depending on a purpose of the clinical examination. One of such widely used methods is a method based on the use of an immunological antigen-antibody reaction.

At present, those known as simplified qualitative method based on the use of the immunological antigen-antibody reaction include an enzyme immunoassay (hereinafter abbreviated as "EIA", if necessary) based on the use of an enzyme as a label, a particle agglutination method based on the use of fine particles, an agglutination inhibition method, and a coloration method based on the use of colored fine particles. Those most universally known as the coloration method are a flow through method and an immuno-affinity chromatography (immuno-affinity chromatography is hereinafter referred to as "immunochromato method", if necessary). A large number of diagnostic reagents based on the use of these methods are commercially available and used for clinical examination. The both methods will be explained below.

The flow through method is a method based on the use of a reactive substance-immobilized membrane obtained by immobilizing a reactive substance 1 (antibody 1) which specifically binds to a substance to be detected on a part of the membrane, and reactive fine particles obtained by immobilizing a reactive substance 2 (antibody 2) which specifically binds to the substance to be detected on the colored particles. The flow through method is based on the following principle. Namely, a complex, in which the substance to be detected is interposed on the membrane between the reactive substance 1 and the reactive substance 2 in a sandwiched manner, is formed by filtrating a specimen containing the substance to be detected and the reactive particles through the reactive substance-immobilized membrane. At this time, a color tone of the fine particles is recognized on the membrane, and thus the presence of the substance to be detected can be confirmed with the naked eye. Those known as the representative flow through method include, for example, a method described in Japanese Patent Application Laid-open No. 63-127160. The flow through method will be specifically explained below as exemplified by a commercially available kit (produced by NIPPON GENE Co., Ltd.) for detecting human luteinizing hormone (hereinafter referred to as "LH", if necessary) in urine. This method also called "immunofilter method" in another name. However, this method is herein called the flow through method.

At first, an anti-LH monoclonal antibody 1 is applied to a nitrocellulose membrane to prepare an antibody-immobilized membrane. On the other hand, an anti-LH monoclonal antibody 2 is immobilized on gold colloid particles having a red-purple color tone. Aliquots, each of which is directed to one time of use, are dispensed and poured into vials, followed by lyophilization to prepare a gold colloid reagent for detection. When a predetermined amount of urine containing LH is added to the vial, the gold colloid reagent for detection is restored by urine to a state of solution to form a complex of (LH)-(anti-LH monoclonal antibody 2)-(gold colloid particle). When the solution of the complex of (LH)-(anti-LH monoclonal antibody 2)-(gold colloid particle) in the vial is added dropwise to the antibody-immobilized membrane, a complex of (membrane)-(anti-LH monoclonal antibody 1)-(LH)-(anti-LH monoclonal antibody 2)-(gold colloid particle) interposing LH in a sandwiched manner is formed during a period of passage of the dropwise-added solution through the membrane in a direction of thickness thereof. At this time, it is possible to confirm red-purple coloration on the membrane with the naked eye. The sandwich-like complex is not formed and thus no coloration appears when no LH is contained in urine. This operation comprises two steps, and it takes about 2 minutes to obtain a decision. Accordingly, it is possible to detect LH in urine conveniently and quickly.

As explained above, the flow through method based on the use of colored fine particles requires a short period of operation time, in which the operation is convenient, and it is easy to make a decision.

The immunochromato method is a method based on the use of a reactive substance-immobilized membrane and reactive fine particles in the same manner as the flow through method. However, the immunochromato method is based on the following principle. Namely, a specimen containing a substance to be detected and the reactive particles are developed on the reactive substance-immobilized membrane in accordance with the same phenomenon as that observed in paper chromatography. When the development flow arrives at a reactive substance-immobilized area on the membrane, a complex is formed, in which the substance to be detected is interposed between a reactive substance 1 (antibody 1) and a reactive substance 2 (antibody 2) in a sandwiched manner. At this time, a color tone of the fine particles is recognized on the membrane, and thus the presence of the substance to be detected can be confirmed with the naked eye. The immunochromato method will be specifically explained below as exemplified by a commercially available pregnancy test agent, Clear Blue One Step-S (produced by Unipas).

A nitrocellulose membrane is cut into strips. Anti-human chorionic gonadotropin (hereinafter referred to as "hCG", if necessary) monoclonal antibody 1 is immobilized on a portion near to one end of the strip to form a reactive area. Next, anti-hCG monoclonal antibody 2-immobilized blue latex particles in a dry state are held at a portion near to the other end opposite to the reactive area in a form of being movable by a development flow. An absorbing pad is allowed to contact with the end of the strip located on the side at which the blue latex particles are held. A urine specimen containing hCG is directly added dropwise to the absorbing pad so that the urine specimen moves from the absorbing pad to the membrane. Further, the urine specimen is allowed to develop on the membrane so that it arrives at the portion at which the blue latex particles are held. At this time, hCG in the development flow binds to an anti-hCG monoclonal antibody 2 on the blue latex particles to form a complex of (hCG)-(anti-hCG monoclonal antibody 2)-(blue latex particle). The complex further continues development to arrive at the reactive area, and hCG in the complex reacts with the anti-hCG monoclonal antibody 1 to form a complex of (membrane)-(anti-hCG monoclonal antibody 1)-(hCG)-(anti-hCG monoclonal antibody 2)-(blue latex particle). At this time, intense blue coloration can be confirmed at the reactive area on the membrane with the naked eye. When hCG is not contained in urine, no intense coloration appears because the sandwich-like complex is not formed at the reactive area. This operation comprises one step of only dropwise adding urine to the absorbing pad, and it takes about 3 minutes to obtain a decision, which is convenient and quick. As described above, in the immunochromato method based on the use of colored fine particles, the operation time is short, the operation is convenient, and it is easy to make a decision.

As described above, the flow through method and the immunochromato method are widely utilized as diagnostic reagents. However, these diagnostic reagents utilize only the combination of the antigen and the antibody. Those based on the use of any in vivo active substance other than the antigen and the antibody are hardly known. Especially, neither diagnostic reagent based on the immunochromato method nor diagnostic reagent based on the flow through method, which uses Ricinus communis agglutinin I (RCA 120), is present.

One of diseases which recently attract attention is rheumatoid arthritis (hereinafter abbreviated as "RA", if necessary). An autoimmune antibody called rheumatoid factor (hereinafter abbreviated as "RF", if necessary) is present in serum of an RA patient. This autoimmune antibody is known to recognize an antigen of an Fc site of human immunoglobulin G (hereinafter abbreviated as "IgG", if necessary). Those adopted as diagnostic methods of clinical chemistry carried out at present include a particle agglutination method based on the use of fine particles obtained by immobilizing an antigen of IgG of an animal such as rabbit, denatured human IgG, or an Fc site thereof, for example, an RA test (diagnostic method for RA for qualitatively detecting RF depending on the presence or absence of agglutination of latex particles on a slide plate), an RAPA method (abbreviation of RA-particle agglutination: method for semiquantitatively determining RF by agglutinating and precipitating latex particles in a microtiter well), and a TIA method (abbreviation of Turbidmetric immunoassay: method for quantitatively determining RF by spectroscopically measuring the degree of agglutination of latex particles). In addition, a method is also adopted, which is based on the use of the rheumatoid factor as a disease marker detected by an EIA method to use an antigen immobilized on a microplate well.

In the case of the diagnosis of RA based on the detection of the rheumatoid factor in accordance with the particle agglutination method and the EIA method carried out at present, an obtained decision is occasionally positive even when the examination is performed by using a serum of a healthy person as a specimen. In addition, an obtained decision is highly frequently positive when the specimen is a serum of a patient suffering from a hepatic disease or a patient suffering from a rheumatism-like disease such as osteoarthritis. Accordingly, the diagnosis methods carried out at present are poor in clinical specificity. Moreover, the positiveness ratio (hereinafter referred to as "clinical sensitivity", if necessary) of these methods with respect to genuine RA patients is not so high. As described above, it is widely recognized that the conventional diagnosis of RA based on the detection of the rheumatoid factor is less useful on the scene of clinical practice in both of the clinical sensitivity and the clinical specificity. However, the foregoing methods are universally used in the present circumstances because there is no other method of clinical chemistry which can be used for diagnosis of RA.

In addition to the problems described above, skilled experience of an examination operator is indispensable for the particle agglutination method in order to make an accurate decision because the subjectivity of the examination operator affects the decision, although the particle agglutination method is composed of the easy operation with a required period of time of about 3 minutes to 2 hours to obtain a result. As for the EIA method, the operation is complicated with a required period of operation time of about 2 hours to 4 hours, in which apparatuses to be exclusively used such as a spectrophotometer and a microplate reader are necessary to make a decision.

Recently, detailed analysis has been made for sugar chains existing at the Fc site of IgG in serum of RA patients. As a result, it has been reported that the galactose content is remarkably decreased in such sugar chains as compared with sugar chains existing at the Fc site of IgG in serum of healthy people [Nature, Vol. 316, p. 452, 1985]. Namely, it has been clarified that the sugar moiety existing at the Fc site of IgG in serum of healthy people is composed of a plurality of sugar chains having mutually different structures, and the existing ratio of sugar chains concerning species is substantially constant among individual members. On the other hand, it has been revealed that the sugar moiety existing at the Fc site of IgG in serum of RA patients is composed of a plurality of sugar chains having mutually different structures, and the existing ratio of sugar chains concerning species is substantially constant among individual members in the same manner as that of healthy people, however, in general, the content of galactose is remarkably decreased. Specifically, it has been reported that three species of sugar chains, which contain 2 moles, 1 mole, and none of galactose respectively, exist at a ratio of about 2:2:1 in the sugar moiety at the Fc site of IgG in serum of healthy people, however, the species of sugar chain, which contains 2 moles of galactose, is remarkably decreased in the sugar moiety at the Fc site of IgG in serum of RA patients, and in general, sugar chains deficient in galactose are greatly increased.

Therefore, based on this fact, there is structural abnormality of sugar chains in the sugar moiety at the Fc site of IgG in serum of RA patients. It has been expected that recognition of the structural abnormality, if possible, may provide a powerful marker for diagnosis of RA.

Accordingly, several diagnostic methods have been proposed, which utilize the structural abnormality concerning sugar chains at the Fc site of IgG in serum of RA patients, in order to replace the conventional methods for RA diagnosis. The proposed methods are roughly classified into two groups in view of objectives of detection. One group resides in a method for utilizing the decrease in galactose content in sugar chains at the Fc site of IgG in serum of RA patients, in which the galactose content in sugar chains at the Fc site of IgG in serum is measured, and an obtained result is compared with the galactose content of healthy people to diagnose the disease as RA when the galactose content of a patient is lower than that of healthy people (Japanese Patent Application Laid-open Nos. 3-73857 and 5-87814). The other group resides in a method based on a viewpoint that an autoantibody against the structural abnormality of sugar chains at the Fc site of IgG, i.e., an anti-galactose-deficient IgG antibody is present in serum of RA patients, in which galactose-deficient IgG is thus previously prepared to measure the anti-galactose-deficient IgG antibody in serum in response thereto (Japanese Patent Application Laid-open No. 3-48700).

Of the two methods described above, the method for measuring the galactose content in sugar chains of IgG in serum has the following drawbacks. Namely, the operation is extremely complicated, and it takes a long reaction time of several hours. In addition, in the case of the method described in Japanese Patent Application Laid-open No. 5-87814, the total IgG amount in serum must be simultaneously measured for each of specimens, and a scintillation counter must be used, which requires complicated data analysis to make a decision. Further, the clinical sensitivity and the clinical specificity of the decision are not satisfactory.

On the other hand, the method for measuring the anti-galactose-deficient IgG antibody is based on the quantitative measurement of the anti-galactose-deficient IgG antibody by using a method similar to EIA, in which the clinical sensitivity and the clinical specificity of the decision are extremely excellent, making it possible to perform reliable diagnosis of RA. However, this method has the following drawbacks. Namely, the operation is complicated because the method is quantitative. It takes 4 hours to overnight to obtain a result. Apparatuses such as a spectrophotometer and a densitometer must be used.

### Disclosure of the Invention

The present invention has been made from a viewpoint as described above, an object of which is to provide a diagnostic reagent and a diagnostic method for diagnosing rheumatoid arthritis accurately, conveniently, and quickly, and provide a detecting reagent for detecting another substance to be detected in which the conventional immunochromato method and the conventional flow through method can be also applied.

As a result of diligent studies by the present inventors in order to achieve the object described above, the present invention has been accomplished by completing a detecting reagent to be used for a detecting method such as the immunochromato method and the flow through method based on the use of Ricinus communis agglutinin I (RCA 120), and completing a detecting reagent and a diagnostic reagent for rheumatoid arthritis for conveniently detecting the anti-galactose-deficient IgG antibody in a body fluid of human by applying the detecting reagent.

Namely, the present invention lies in a detecting reagent for detecting a substance to be detected having a sugar chain containing galactose and/or β-N-acetylgalactosamine at its terminal, the detecting reagent comprising a membrane on which a first reactive substance is immobilized and fine particles on which a second reactive substance is immobilized, wherein one of the reactive substances is Ricinus communis agglutinin I, and the other of the reactive substances is a reactive substance which specifically binds to the substance to be detected, and has no sugar chain containing galactose and β-N-acetylgalactosamine at its terminal.

In another aspect, the present invention provides a detecting reagent for detecting an anti-galactose-deficient IgG antibody, the detecting reagent comprising a membrane and fine particles, wherein Ricinus communis agglutinin I is immobilized on one of the membrane and the fine particles, and galactose-deficient IgG is immobilized on the other.

In still another aspect, the present invention provides a diagnostic agent for rheumatoid arthritis comprising the detecting reagent described above. In still another aspect, the present invention provides a method for detecting rheumatoid arthritis, comprising the steps of:
reacting a membrane on which one of galactose-deficient IgG and Ricinus communis agglutinin I is immobilized, colored fine particles on which the other is immobilized, and a biological sample collected from a test subject with each other; and
detecting the colored fine particles captured on the membrane through binding between an anti-galactose-deficient IgG antibody contained in the sample and the galactose-deficient IgG and binding between Ricinus communis agglutinin I and galactose contained in the anti-galactose-deficient IgG antibody.

The present invention will be explained in detail below.

### 〈1〉 Detecting reagent for detecting substance to be detected

The detecting reagent for detecting a substance to be detected of the present invention is provided for detecting a substance to be detected having a sugar chain containing galactose and/or β-N-acetylgalactosamine at its terminal, the detecting reagent comprising a membrane on which a first reactive substance is immobilized and fine particles on which a second reactive substance is immobilized. One of the reactive substances is Ricinus communis agglutinin I, and the other of the reactive substances is a reactive substance which specifically binds to the substance to be detected, and has no sugar chain containing galactose and β-N-acetylgalactosamine at its terminals.

The detecting reagent utilizes specific binding between galactose or β-N-acetylgalactosamine and Ricinus communis agglutinin I which specifically binds thereto for the purpose of use in the flow through method and the immunochromato method, wherein the substance to be detected is qualitatively detected by detecting colored fine particles captured on the membrane through binding between the substance to be detected having a sugar chain containing galactose or β-N-acetylgalactosamine contained in a sample and Ricinus communis agglutinin I, and binding between the substance to be detected and the reactive substance which specifically binds to the substance to be detected, and has no sugar chain containing galactose and β-N-acetylgalactosamine.

The detecting reagent of the present invention will be described in detail below.

### (1) Fine particles

The fine particles are used as a label for deciding the presence of the substance to be detected with the naked eye, and are preferably colored in order to facilitate the decision with the naked eye. Materials for the fine particles include, for example, water insoluble materials such as homogeneous spherical particles composed of synthetic polymers such as polystyrene latex or natural polymers such as gelatin, or metal colloid particles such as gold colloid. Coloration is unnecessary for the metal colloid particles because they have an intrinsic color. Those having no color may be appropriately colored by using a coloring matter. The fine particles should have a particle size smaller than a pore size of the membrane. In general, the usable particle size is in a range of 0.01 to 5 µm. Especially preferably, the particle size is about 0.05 to 2 µm.

### (2) Membrane

The membrane used in the present invention is provided for indirectly capturing the fine particles through the binding between the substance to be detected and Ricinus communis agglutinin I. Any membrane may be used provided that the reactive substance can be immobilized on the membrane, unreacted fine particles are allowed to pass through the membrane in the case of the flow through method, and fine particles can be developed in an aqueous solution by using the membrane as a chromatography carrier in the case of the immunochromato method. Materials for the membrane include, for example, porous membranes having three-dimensional structures such as nylon membranes and nitrocellulose membranes, including any of synthetic and natural polymer membranes. The pore size of the membrane may be a size at which the reactive fine particles can pass through without causing clogging. Especially preferably, the pore size is in a range of 0.2 to 8 µm.

### (3) First and second reactive substances

One of the first and second reactive substances is Ricinus communis agglutinin I (hereinafter referred to as "RCA 120"). RCA 120 is a lectin originating from castor bean (Ricinus communis), which has an activity to specifically bind to galactose and β-N-acetylgalactosamine. RCA 120 can be obtained, for example, by means of purification from castor bean in accordance with a method of J. U. Baenziger et al. (J. Biol. Chem., Vol. 254, pp. 9795-9799, 1979). Commercially available RCA 120 may be used. Lectins usable in the present invention other than RCA 120, which specifically bind to galactose or β-N-acetylgalactosamine, include, for example, Ricinus communis agglutinin II (RCA 60), beetle agglutinin (Allo A), mushroom lectin (ABA), and peanut lectin (PNA). Among them, RCA 120 is especially preferred.

The other of the first and second reactive substances is a substance which makes it possible to detect the substance to be detected in combination with RCA 120. It is necessary for this reactive substance not to bind to RCA 120. Therefore, it is necessary to have no sugar chain containing galactose or β-N-acetylgalactosamine at its terminal. This reactive substance is hereinafter referred to as "galactose-deficient reactive substance". Specifically, the galactose-deficient reactive substance includes, for example, galactose-deficient IgG, galactose-deficient antibodies against glycoproteins comprising sugar chains containing galactose and/or β-N-acetylgalactosamine.

### (4) Immobilization of respective reactive substances onto fine particles and membrane

The method for immobilizing the first and second reactive substances onto the fine particles and the membrane may be a physical adsorption method, a chemical binding method, and any one of other methods. Namely, any immobilizing means may be used provided that the reactive substance such as RCA 120 is not disengaged from the fine particles and the membrane.

In the following description, the membrane on which the reactive substance is immobilized is referred to as "reactive substance-immobilized membrane", and the fine particles on which the reactive substance is immobilized are referred to as "reactive fine particles".

### (5) Substance to be detected

The substance to be detected by the detecting reagent of the present invention is a substance having a sugar chain containing galactose and/or β-N-acetylgalactosamine at its terminal, and the substance specifically binds to RCA 120. Specifically, the substance is an anti-galactose-deficient IgG antibody, or a glycoprotein having a sugar chain containing galactose and/or β-N-acetylgalactosamine at its terminal. Such a glycoprotein includes, for example, thyroglobulin and transferrin.

When the substance to be detected is the anti-galactose-deficient IgG antibody, the galactose-deficient reactive substance is galactose-deficient IgG. When the substance to be detected is the glycoprotein, the galactose-deficient reactive substance is the galactose-deficient antibody against the glycoprotein.

### (6) Detecting reagent and method of using the same

The detecting reagent based on the use of the foregoing materials and the method of using it will be explained below. The following explanation will be made as exemplified by the use of the flow through method. However, the present detecting reagent may be also used in accordance with the immunochromato method. The immunochromato method can be carried out by conforming the shape of the membrane and the site of immobilization of the reactive substance on the membrane to the immunochromato method, and holding the reactive fine particles on the membrane in a dry state.

The detecting reagent comprises the membrane on which the first reactive substance is immobilized, and the fine particles on which the second reactive substance is immobilized. The detecting reagent optionally comprises a reaction vessel, a solution for diluting a specimen, and a washing solution.

In order to maintain the colloid-chemical stability and the specific binding activity of the reactive substance, the reactive particles obtained as described above are suspended in a buffer or the like containing bovine serum albumin of about 0.05 to 3% (w/w), a water-soluble polymer such as polyethylene glycol of about 0.05 to 10% (w/w), other stabilizers, and a preservative, and stored at a cold place. For the purpose of storage for a long period of time, the suspension may be lyophilized to be used by dissolving the contents with distilled water or the like upon examination.

On the other hand, in order to avoid nonspecific adsorption of a specimen or the reactive fine particles, the reactive substance-immobilized membrane is subjected to a blocking treatment with bovine serum albumin or the like of about 0.05 to 5% (w/w) and stored under a dry condition.

### [Reaction vessel]

When the detecting reagent of the present invention is used in accordance with the flow through method, it is preferred that the flow rate and the flow amount of the reagent and the specimen relevant to the reaction are stable, and their use is easy. Therefore, a reaction vessel is necessary. The reaction vessel is composed of a material such as plastic, having a window arranged for adding the respective reagents and the specimen into the vessel dropwise. The reaction vessel comprises a molded vessel, an absorbing member, a reactive substance-immobilized membrane, and other necessary members.

The absorbing member is preferably made of a material having performance capable of sufficiently absorbing each of the added reagents used in the test without any variation in the absorbing speed of each of the added reaction reagents and the specimen. Those preferably used as such a material include, for example, cotton, nonwoven fabric, filter paper, and porous plastic. However, any material may be used provided that it has the performance described above. The absorbing member may have any size provided that the respective reaction reagents and the specimen added upon the test are completely absorbed.

In the reaction vessel, the reactive substance-immobilized membrane is placed on the absorbing member in the vessel, and they are fixed to the vessel in order to avoid movement in the vessel. At this time, the position for fixing the reactive substance-immobilized membrane and the absorbing member is conformed to the window through which the respective reaction reagents and the specimen are added dropwise. Tissue, mesh or the like for making complete contact, and filter paper or the like for controlling the absorbing speeds of the respective reagents and the specimen may be interposed between the absorbing member and the reactive substance-immobilized membrane.

### [Other reagents (solution for diluting specimen and washing solution)]

When the detection is carried out by using the reagent of the present invention, a specimen may be directly used without dilution. However, the specimen has to be diluted when the semiquantitative examination is performed, or when any necessity arises depending on the material, production method, and properties of the reactive substance-immobilized membrane and the reactive fine particles. Those appropriately usable as a solution for washing the specimen in such a case include a physiological salt solution, various types of buffers, and solutions obtained by adding a surfactant or bovine serum albumin of about 0.05 to 3% (w/w) to the foregoing solutions. In the flow through method to which the present invention is applied, a physiological salt solution, various types of buffers, and solutions obtained by adding a surfactant or bovine serum albumin of about 0.05 to 3% (w/w) to the foregoing solutions may be appropriately used as well for the washing solution which is used after the dropwise addition of the specimen and the suspension of the reactive fine particles, if necessary.

### [Detection kit]

A detection kit may be provided by incorporating necessary solutions and reagents into one package in order to make the detection of the substance to be detected according to the present invention without using special apparatuses and without preparing solutions and reagents by a user. Specifically, one package of the detection kit is provided, which package includes sets corresponding to an arbitrary number of measurement times, each of the sets being prepared by combining an operating manual, a suspension of reactive fine particles, a reaction vessel including a reactive substance-immobilized membrane incorporated therein, and optionally a solution for diluting a specimen and a washing solution.

### [Method of use]

A detection kit is provided, which kit comprises as the detecting reagent of the present invention, comprising the suspension of the reactive fine particles on which RCA 120 is immobilized, the reaction vessel including the reactive substance-immobilized membrane incorporated therein on which the galactose-deficient reactive substance is immobilized, the solution for diluting a specimen, and the washing solution. A method of the use of the detection kit in accordance with the flow through method will be explained below.

A specimen is added dropwise (directly or after dilution with the solution for diluting the specimen) to the reactive substance-immobilized membrane through the window of the reaction vessel, and is completely absorbed by the absorbing member. After that, the suspension of the reactive fine particles on which RCA 120 is immobilized is added dropwise to the reactive substance-immobilized membrane, and is absorbed by the absorbing member. The presence or absence of coloration on the membrane is observed with the naked eye. When the substance to be detected, which contains a sugar chain containing galactose and/or β-N-acetylgalactosamine and specifically binds to the galactose-deficient reactive substance, is present in the specimen, then the substance to be detected binds to the galactose-deficient reactive substance immobilized on the membrane, and RCA 120 binds to galactose or β-N-acetylgalactosamine contained in the substance to be detected. As a result, the fine particles are captured on the membrane.

Therefore, when the coloration with the reactive fine particles on which RCA 120 is immobilized is recognized on the membrane, then the substance to be detected is present in the specimen, and thus it is possible to make a positive decision. When the coloration is not recognized, then the substance to be detected is absent, and thus it is possible to make a negative decision. However, in a competitive reaction, when the coloration is not recognized, a positive decision may be made in some cases. The process from the dropwise addition of the specimen to the decision of presence or absence of coloration can be performed during a period of about 30 seconds to 10 minutes. The operation comprises two steps. The washing solution may be added dropwise after the dropwise addition of the specimen and after the dropwise addition of the suspension of the reactive fine particles. In such a case, the operation comprises three or four steps. The membrane after the decision can be dried and stored for a long period of time without any change of the coloration.

In the present invention, the flow through method is an especially effective method for a detection system which requires B/F separation (physical separation of bound type (Bound, B) generated by binding of an antibody from free type (Free, F) without binding of the antibody in an antigen-antibody reaction). However, in the case of a detection system which requires no B/F separation, i.e., a detection system which contains substantially no existing contaminant that affects the specific binding between RCA 120 and the substance to be detected, the present invention can be carried out as a one-step assay method in which the reactive fine particles are immobilized on an upper portion of the membrane in a dry state, and the reactive fine particles are restored to a solution state by means of an operation of dropwise addition of a specimen to advance the reaction. Alternatively, the present invention can be also carried out in accordance with the immunochromato method which uses a test sheet obtained by cutting the membrane into strips, and making the reactive substance immobilized at one end of each strip and the reactive fine particles held at the other end in a dry state.

### 〈2〉 Detecting reagent for anti-galactose-deficient IgG antibody

The detecting reagent for the anti-galactose-deficient IgG antibody of the present invention comprises the membrane and the fine particles. RCA 120 is immobilized on one of the membrane and the fine particles, and the galactose-deficient IgG is immobilized on the other.

The membrane, the fine particles, RCA 120, the reactive fine particles, the reactive substance-immobilized membrane, the reaction vessel, the solution for diluting a specimen, and the washing solution are provided in the same manner as described in the foregoing item 〈1〉. The detecting reagent of the this embodiment is characterized in that the substance to be detected is the anti-galactose-deficient IgG antibody, and one of the reactive substances is the galactose-deficient IgG.

The detecting reagent for the anti-galactose-deficient IgG antibody and the detecting method based on the use of the reagent according to the present invention will be explained as exemplified by the flow through method. A blood sample, which is an ordinary specimen, requires the B/F separation because of the presence of large amounts of, for example, immunoglobulin, glycoproteins, and polysaccharides other than the anti-galactose-deficient IgG antibody. Upon the use of the detecting reagent of the present invention, it is preferable to utilize the flow through method. The respective reaction reagents are prepared by the respective preparation methods in accordance with the foregoing item 〈1〉.

### [Suspension of RCA 120-immobilized fine particles]

RCA 120 as the reactive substance is immobilized on colored fine particles to prepare a suspension of RCA 120-immobilized fine particles.

### [Galactose-deficient IgG-immobilized membrane]

Galactose-deficient IgG is used as the reactive substance to prepare a galactose-deficient IgG-immobilized membrane. Galactose-deficient IgG is obtained such that human IgG is subjected to an enzymatic degalactosylation treatment, followed by purification in accordance with a known method (see Japanese Patent Application Laid-open No. 3-48700 or 5-87814). Substantial removal of galactose is confirmed by means of, for example, ion exchange chromatography. Next, the obtained galactose-deficient IgG is immobilized on a membrane such as a membrane filter in accordance with a technique such as spotting, spray, or printing to prepare a galactose-deficient IgG-immobilized membrane. The amount of immobilization can be appropriately adjusted depending on, for example, the sensitivity of a finally obtained detecting reagent. However, the amount of immobilization is especially preferably 0.5 to 10 µg.

### [Reaction vessel]

The absorbing member and the galactose-deficient IgG-immobilized membrane are incorporated into a vessel made of plastic in the same manner as described above to prepare a reaction vessel.

### [Anti-galactose-deficient IgG antibody detection kit]

A detection kit may be provided by incorporating necessary solutions and reagents into one package in order to carry out the detection of the anti-galactose-deficient IgG antibody according to the present invention without using special apparatuses and without preparing solutions and reagents by a user. Specifically, one package of the anti-galactose-deficient IgG detection kit is provided, which package includes sets corresponding to an arbitrary number of measurement times, each of the sets being prepared by combining an operating manual, a suspension of the RCA 120-immobilized fine particles, a reaction vessel including the galactose-deficient IgG-immobilized membrane incorporated therein, and optionally a solution for diluting a specimen and a washing solution.

### [Method for using detecting reagent for anti-galactose-deficient IgG antibody]

The detection of the anti-galactose-deficient IgG antibody is carried out as follows by using the detecting reagent for the anti-galactose-deficient IgG antibody.

A specimen is added dropwise (directly or after dilution with the solution for diluting the specimen) to the galactose-deficient IgG-immobilized membrane through the window of the reaction vessel, and is completely absorbed by the absorbing member. After that, the suspension of the RCA 120-immobilized fine particles is added dropwise to the galactose-deficient IgG-immobilized membrane, and is absorbed by the absorbing member. The presence or absence of coloration on the membrane is observed with the naked eye. When the coloration with the RCA 120-immobilized fine particles is recognized, then the anti-galactose-deficient IgG antibody is present in the specimen, and thus a positive decision is made. When the coloration is not recognized, then the anti-galactose-deficient IgG antibody is absent, and thus a negative decision is made. The specimen (biological sample), for which the anti-galactose-deficient IgG antibody-detecting reagent is usable, includes, for example, blood (whole blood), serum, blood plasma, saliva, and synovial fluid.

### [Reaction principle]

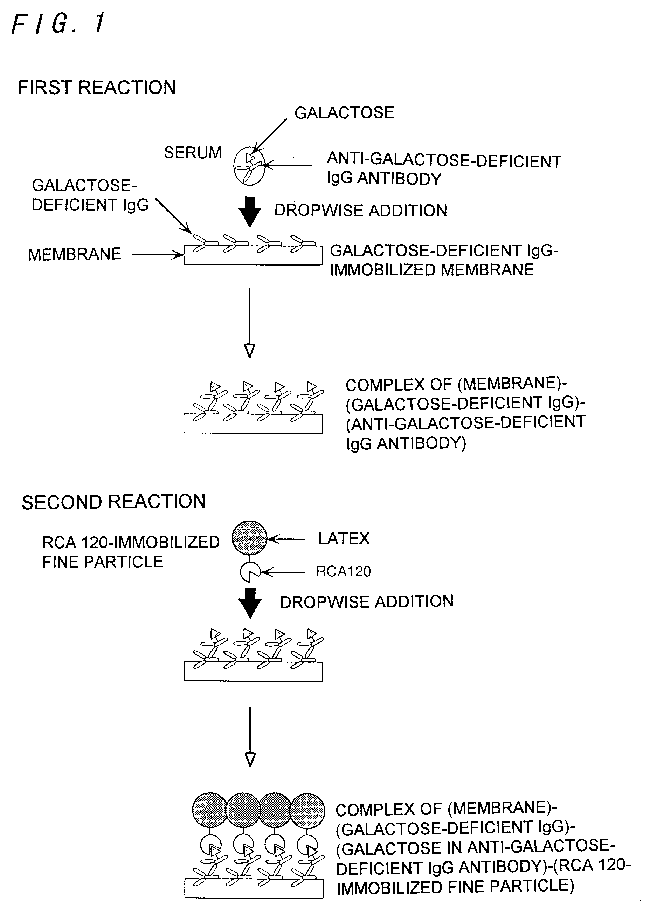

The reaction principle in the use of the anti-galactose-deficient IgG antibody-detecting reagent will be described below with reference to Fig. 1. In Fig. 1, the anti-galactose-deficient IgG antibody is conveniently illustrated as one of the IgG type. However, the antibody is not limited to those of the IgG type. An antibody of another type is allowable.

In the first reaction, the specimen is added dropwise to the galactose-deficient IgG-immobilized membrane. The anti-galactose-deficient IgG antibody in the specimen makes an antigen-antibody reaction with the galactose-deficient IgG on the membrane to form a complex of (membrane)-(galactose-deficient IgG)-(anti-galactose-deficient IgG antibody).

In the second reaction, when the RCA 120-immobilized fine particles are added dropwise to the membrane, RCA 120 binds to galactose in a sugar chain existing in the molecule of the anti-galactose-deficient IgG antibody in the complex of (membrane)-(galactose-deficient IgG)-(anti-galactose-deficient IgG antibody) to form a complex of (membrane)-(galactose-deficient IgG)-(galactose in anti-galactose-deficient IgG antibody)-(RCA 120-immobilized fine particle). The color of the fine particles can be confirmed apparently as coloration on the membrane with the naked eye. When the substance to be detected is the anti-galactose-deficient IgG antibody in serum of a patient of rheumatoid arthritis, especially an antibody of the IgG type, the antibody is mostly deficient in galactose. However, all antibodies of the IgG type are not completely deficient in galactose. Galactose remains in some antibodies, and hence it is possible to make binding to RCA 120.

When the anti-galactose-deficient IgG antibody is not present in a specimen, then RCA 120 does not bind to the galactose-deficient IgG, and the complex in the first reaction is not formed. Accordingly, the RCA 120-immobilized fine particles pass through the membrane, and no coloration is confirmed. According to the principle of this method, the intensity of the shade of coloration varies depending on the amount of the anti-galactose-deficient IgG antibody in a specimen. Accordingly, the amount of the anti-galactose-deficient IgG antibody in the specimen can be quantitatively determined as well by measuring the intensity of coloration by using a densitometer, a color-difference meter or the like.

The detecting reagent for the anti-galactose-deficient IgG antibody is provided as described above. The anti-galactose-deficient IgG antibody in a specimen can be detected qualitatively or semiquantitatively as well by using the detecting reagent in a short period of time of 0.5 to not more than 10 minutes in accordance with the simple operation comprising two to four steps more conveniently as compared with the conventional quantitative method.

### 〈3〉 Diagnostic reagent for rheumatoid arthritis (RA)

The anti-galactose-deficient IgG antibody in a specimen can be detected reliably as described above by using the detecting reagent for the anti-galactose-deficient IgG antibody according to the present invention. It is described in Japanese Patent Application Laid-open No. 3-48700 that a method for quantitatively determining the anti-galactose-deficient IgG antibody makes it possible to perform accurate diagnosis of rheumatoid arthritis. In view of the facts described above, when the detecting reagent for the anti-galactose-deficient IgG antibody according to the present invention is applied to a diagnostic objective of rheumatoid arthritis, it is possible to perform accurate diagnosis of rheumatoid arthritis in the same manner as performed by the quantitative method of the invention described in Japanese Patent Application Laid-open No. 3-48700.

Therefore, the detecting reagent for the anti-galactose-deficient IgG antibody according to the present invention can be used as a diagnostic reagent for rheumatoid arthritis (RA).

When the detecting reagent for the anti-galactose-deficient IgG antibody is used as a diagnostic reagent for rheumatoid arthritis (RA), it is possible to perform diagnosis of rheumatoid arthritis more accurately, conveniently, and quickly as compared with the conventional agglutination method as well as the quantitative method of the invention described in Japanese Patent Application Laid-open No. 3-48700. The detecting reagent for the anti-galactose-deficient IgG antibody according to the present invention is useful as a diagnostic method for rheumatoid arthritis.

### 〈4〉 Method for detecting rheumatoid arthritis

Rheumatoid arthritis can be detected by using the diagnostic agent for rheumatoid arthritis. Namely, the method for detecting rheumatoid arthritis according to the present invention comprises the steps of:
reacting a membrane on which one of galactose-deficient IgG and RCA 120 is immobilized, colored fine particles on which the other is immobilized, and a biological sample collected from a test subject with each other; and
detecting the colored fine particles captured on the membrane through binding between an anti-galactose-deficient IgG antibody contained in the sample and the galactose-deficient IgG and binding between Ricinus communis agglutinin I and galactose contained in the anti-galactose-deficient IgG antibody.

The present invention will be described in detail with reference to Test Example described below.

### (Test Example 1)

This test was performed in order to compare the detecting method based on the use of the detecting reagent of the present invention (the present method) with the conventional quantitative method described in Examples 1 to 3 described in Japanese Patent Application Laid-open No. 3-48700 (the conventional method).

### (1) Sample specimen

Those used as sample specimens were 17 specimens in total, of 6 specimens of standard serums from rheumatoid arthritis (RA) patients [Rheumatoid Factor Control/Calibrator: produced by INTERNATIONAL ENZYMES, INC., catalog No.: 7112, 7113, 7114, 7115, 7116, 7117], 8 clinical specimens from rheumatoid arthritis (RA) patients, and 3 specimens of serums from healthy people.

### (Test method)

### (i) Detecting method based on use of detecting reagent of the present invention (hereinafter referred to as "the present method")

A detecting reagent for the anti-galactose-deficient IgG antibody was prepared in accordance with Example 1 described later on. The anti-galactose-deficient IgG antibody was qualitatively detected by using the prepared detecting reagent. A positive or negative decision was made for rheumatoid arthritis (RA) depending on the presence or absence of a red spot recognized through a window of a reaction vessel.

### (ii) Conventional method

The anti-galactose-deficient IgG antibody in a specimen was quantitatively determined in accordance with a method similar to EIA. The method similar to EIA was operated by using a microtiter well made of plastic with the galactose-deficient IgG previously immobilized thereon and subjected to a blocking treatment. The specimen was diluted, and an aliquot (100 µl) was added to the well to perform incubation for 2 hours. After washing, biotin-labeled RCA 120 was added to perform incubation for further 1 hour. After washing, avidin-labeled horseradish peroxidase was added to perform incubation for 1 hour. After washing, the substrate was reacted for 30 minutes to cause color development. The density of color development in the microtiter well was measured by using a commercially available microplate reader (produced by Bio Rad Laboratories). The absorbance was multiplied by a dilution ratio to obtain a quantitatively measured value. A cutoff value for measured values obtained by the quantitative method was set at 100. Those having values less than 100 were decided as rheumatoid arthritis (RA) negative, and those having values not less than 100 were decided as positive.

The 17 specimens described above were used to test the respective specimens in accordance with the present method described in the item (i) and the conventional method described in the item (ii). Specifically, the respective specimens were decided to be rheumatoid arthritis (RA) positive or negative in accordance with the present method. After that, the respective specimens having been classified into respective groups each decided to be rheumatoid arthritis (RA) positive or negative in accordance with the present method were tested in accordance with the conventional method whether the respective specimens were decided to be rheumatoid arthritis (RA) positive or negative.

### (3) Test result

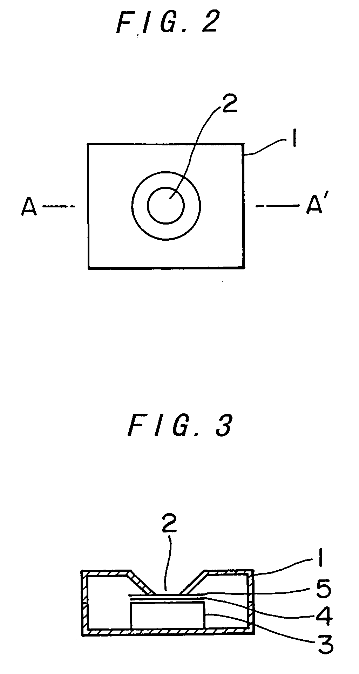

Results of this test are shown in Table 1. Table 1 was completed with reference to Fig. 3-4 on page 59 in "Clinical Epidemiology" written by Robert H. Fletcher et al. (translated by Shigeru Hisamichi et al., Igaku-Syoin, 1986). The group decided to be rheumatoid arthritis (RA) positive in accordance with the present method is described on the first line, in which the number of specimens also decided to be positive in accordance with the conventional method is described in the first column, and the number of specimens decided to be negative in accordance with the conventional method is described in the second column. Similarly, the group decided to be rheumatoid arthritis (RA) negative in accordance with the present method is described on the second line, in which the number of specimens decided to be positive in accordance with the conventional method is described in the first column, and the number of specimens also decided to be negative in accordance with the conventional method is described in the second column. The total of the results of decision for rheumatoid arthritis (RA) in accordance with the present method is summarized and described in the third column, and the total of the results of decision for rheumatoid arthritis (RA) in accordance with the conventional method is summarized and described on the third line.

Table 1 clarifies a result of comparison between the results of decision on whether the specimen is rheumatoid arthritis (RA) positive or negative obtained in accordance with the present method described in the item (i) and the result of decision obtained in accordance with the conventional method described in the item (ii). Those which are rheumatoid arthritis (RA) positive in accordance with the present method are also positive in accordance with the conventional method as they are. Those which are negative in accordance with the present method are also negative in accordance with the conventional method as they are. The results of decision were coincident with each other at a ratio of 100%.

The operation of the conventional method described in the item (ii) was complicated, which took as long as 4.5 hours. On the contrary, the detecting reagent for the anti-galactose-deficient IgG antibody in accordance with the detecting method (the present method) by using the detecting reagent of the present invention required the number of operation steps of two, only comprising the steps of the dropwise addition of the specimen and the dropwise addition of the suspension of the RCA 120-immobilized fine particles, in which the operation was simple and convenient, and the operation was quick requiring a period of time of 2 minutes from the dropwise addition of the specimen to the decision.

According to the results described above, the decision on whether the specimen was rheumatoid arthritis (RA) positive or negative obtained by using the detecting reagent for the anti-galactose-deficient IgG antibody of the present invention was well coincident with the decision obtained by using the conventional quantitative method. Accordingly, it is clear that the reagent of the present invention detects the anti-galactose-deficient IgG antibody in human serum accurately and quickly. Therefore, it has been revealed that the diagnosis of rheumatoid arthritis (RA) can be carried out accurately, conveniently and quickly.

**Table 1**

| Present method | Conventional method | | |
|---|---|---|---|
| | Positive | Negative | Total |
| Positive | 14 | 0 | 14 |
| Negative | 0 | 3 | 3 |
| Total | 14 | 3 | 17 |

### Brief Description of Drawings

Fig. 1 conceptually shows the reaction principle of the present invention.

Fig. 2 shows a plan view illustrating a detecting reagent according to an embodiment of the present invention.

Fig. 3 shows a cross-sectional view taking along a plane A-A' of the detecting reagent shown in Fig. 2.

### Best Mode for Carrying Out the Invention

Examples will be described below in order to more specifically explain the present invention. However, the present invention is not limited to the following examples.

### Example 1: Detecting Reagent for Anti-Galactose-Deficient IgG Antibody to Be Used for Flow Through Method Based on Use of RCA 120, and Detecting Method by Using the Same

### 〈1〉 Preparation of galactose-deficient IgG

Galactose-deficient IgG was prepared as follows in accordance with a known method (Japanese Patent Application Laid-open No. 3-48700).

Human IgG protein (10 mg/ml) was subjected to a sialidase treatment (500 mU/ml) in 0.1 M acetate buffer (pH 5.0), and 0.1 M citrate-phosphate buffer (pH 7.0) was added thereto to perform a treatment with β-galactosidase (100 mU/ml). After the enzyme treatments, glycine buffer (1.5 M glycine-HCl, 3 M sodium chloride, pH 8.9; hereinafter referred to as "binding buffer", if necessary) in an amount ten times the amount of the enzyme treatment solution was added. In order to remove BSA, sialidase, β-galactosidase, and other components contained in the enzyme treatment solution, Protein G-Sepharose CL-4B was used to purify galactose-deficient IgG from the enzyme treatment solution. Specifically, the enzyme-treated sample was applied to Protein G-Sepharose CL-4B (1 × 7.8 cm) previously equilibrated with the binding buffer, and the column was sufficiently washed with the binding buffer. After that, galactose-deficient IgG was recovered by using 0.1 M glycine-HCl (pH 3.0). Upon the recovery, in order to prevent galactose-deficient IgG from being left in a state of pH 3.0 for a long period of time, the binding buffer in the same amount as a fractionating volume was previously added to fraction tubes for recovering galactose-deficient IgG. After the recovery, galactose-deficient IgG was sufficiently dialyzed against phosphate buffer (10 mM phosphate, 0.15 M sodium chloride, pH 7.2).

### 〈2〉 Method for preparing galactose-deficient IgG-immobilized membrane

Galactose-deficient IgG (5 mg/ml) prepared in the foregoing item 〈1〉 was spotted and immobilized (1 µg) on a nitrocellulose membrane having a pore size of 3 µm (produced by ADVANTEC TOYO) by using a micropipette. The membrane was sufficiently dried. In order to avoid nonspecific adsorption in the downstream process, blocking was performed by using blocking buffer (50 mM Tris-HCl, 0.15 M sodium chloride, 0.1% BSA (w/w), pH 8.0; hereinafter simply referred to as "blocking buffer"), followed by sufficient drying to obtain a galactose-deficient IgG-immobilized membrane.

### 〈3〉 Method for preparing RCA 120-immobilized fine particles

RCA 120 (2.5 mg/ml, produced by HONEN CORPORATION) was diluted with Tris buffer (50 mM Tris-HCl, pH 8.0) to a concentration of 1 mg/ml. Red carboxylated latex particles having a particle size of 0.3 µm (100 µl, solid content concentration: 10%, produced by Japan Synthetic Rubber Co., Ltd.) were added to 0.9 ml of the RCA 120 solution, and the mixture was agitated at 45°C for 60 minutes, followed by centrifugation (at 8,000 rpm for 30 minutes) to obtain a residual precipitate. The precipitate was dispersed by adding 1 ml of Tris buffer described above, followed by centrifugation again (at 8,000 rpm for 30 minutes) to obtain a residual precipitate. The precipitate was dispersed in 200 ml of Tris buffer (50 mM Tris-HCl, pH 8.0) containing 0.1% BSA, 0.15 M sodium chloride, and 0.02% sodium azide to obtain a suspension of the RCA 120-immobilized fine particles.

### 〈4〉 Assembly of reaction vessel

An absorbent cotton pad 3 cut into an appropriate size was put into a plastic vessel 1 having a cubic shape of 2.5 cm × 2.5 cm × 1.0 cm with a circular window 2 having a diameter of 0.5 cm opened at a portion for reagent addition. A sheet of filter paper No. 6 (produced by ADVANTEC TOYO) cut into a square of 1 cm indicated by a reference numeral 4 was placed on the absorbent cotton pad 3. The galactose-deficient IgG-immobilized membrane 5 cut into a square of 1 cm was fixed thereon with a spotted portion of galactose-deficient IgG arranged to oppose the window of the vessel. Thus a reaction vessel was obtained (Figs. 2 and 3).

### 〈5〉 Assembly of detecting reagent for anti-galactose-deficient IgG antibody

A detecting reagent for the anti-galactose-deficient IgG antibody was obtained by assembling the suspension of the RCA 120-immobilized fine particles and the reaction vessel into which the galactose-deficient IgG-immobilized membrane had been incorporated.

### 〈6〉 Practice of detecting method based on use of detecting reagent for anti-galactose-deficient IgG (flow through method)

A serum of a healthy person and a standard serum of an RA patient [Rheumatoid Factor Control/Calibrator, produced by INTERNATIONAL ENZYMES. INC., catalog No. 7112] were collected with a micropipette (each 100 µl), and each added dropwise through the window of the reaction vessel of the detecting reagent for the anti-galactose-deficient IgG antibody. The samples were completely absorbed, immediately after which the suspension of the RCA 120-immobilized fine particles (200 µl) of the detecting reagent for the anti-galactose-deficient IgG antibody was added dropwise to the respective reaction vessels, and the suspension was absorbed. After that, the presence or absence of coloration was confirmed with the naked eye. As a result, the white color of the nitrocellulose membrane did not change at all as viewed through the window of the reaction vessel to which the serum of the healthy person had been added dropwise. Thus no coloration was recognized, and the sample was decided to be negative.

On the other hand, a red spot was clearly recognized through the window of the reaction vessel to which the RA standard serum had been added dropwise. Thus the sample was decided to be positive. The operation consisted of two steps of the dropwise addition of the specimen and the dropwise addition of the suspension of the RCA 120-immobilized fine particles, which was extremely simple and convenient. The operation was quick, requiring a period of time of 2 minutes from the dropwise addition of the specimen to the decision.

As described above, the present invention provides the detecting reagent for the anti-galactose-deficient IgG antibody to be used for the flow through method based on the use of RCA 120.

### Example 2: Detecting Reagent for Thyroglobulin to Be Used for Immunochromato Method Based on Use of RCA 120, and Detecting Method by Using the Same

### 〈1〉 Method for preparing galactose-deficient anti-thyroglobulin monoclonal antibody

The process was carried out in accordance with the method described in the item 〈1〉 in Example 1 provided that the human IgG protein was replaced with a 1/10 amount of an anti-thyroglobulin monoclonal antibody. Namely, the anti-thyroglobulin monoclonal antibody [Anti Thyroglobulin (Mono), produced by Biomeda, 1 mg] was treated with sialidase and β-galactosidase to prepare a galactose-deficient anti-thyroglobulin monoclonal antibody.

### 〈2〉 Method for preparing galactose-deficient anti-thyroglobulin monoclonal antibody-immobilized gold colloid

A suspension was prepared by diluting gold colloid (Gold colloid, Em GC 10, produced by British Biocell International) with Tris buffer (50 mM Tris-HCl, pH 8.0) and adjusting its absorbance to 1.0 as measured by using an optical path length of 1 cm at 540 nm. The prepared suspension (20 ml) was mixed with the galactose-deficient anti-thyroglobulin monoclonal antibody (0.9 mg) prepared in the foregoing item 〈1〉, followed by agitation at 45°C for 60 minutes. The mixture was centrifuged at 50,000 × g for 20 minutes. A supernatant was carefully removed, and a red-purple residual precipitate in a pellet form was suspended in 20 ml of Tris buffer described above. The suspension was further centrifuged under the same condition as described above. A residual precipitate was suspended again with Tris buffer described above (5 ml) containing 0.1% BSA and 10% sucrose to obtain a suspension of galactose-deficient anti-thyroglobulin monoclonal antibody-immobilized gold colloid.

### 〈3〉 Method for preparing test sheet

A nitrocellulose membrane having a pore size of 3 µm (produced by ADVANTEC TOYO) was cut into strips having a width of 0.5 cm and a length of 6 cm. RCA 120 (2.5 mg/ml, produced by HONEN CORPORATION) was spotted (1 µg) with a micropipette at a position distant 1 cm from an end of the strip, followed by air-drying. In order to avoid any influence of nonspecific adsorption in the downstream process, the strip was immersed in the blocking buffer. The strip was sufficiently dried. Tris buffer described above (200 µl) containing 30% sucrose was spotted at a position distant 1 cm from the other end of the strip opposite to the portion of application of RCA 120, followed by air-drying. The suspension of galactose-deficient anti-thyroglobulin monoclonal antibody-immobilized gold colloid (50 µl) was spotted on the portion of application of sucrose, followed by air-drying to obtain a test sheet.

### 〈4〉 Assembly of detecting reagent for thyroglobulin

A celluloid plate was cut into strips having a width of 0.5 cm and a length of 6 cm. The test sheet described above was superimposed on the celluloid strip to make adhesion with an adhesive or an adhesive double coated tape. A sheet of filter paper cut to have a width of 0.5 cm and a length of 2 cm to be used as an absorbing pad for holding a specimen was adhered to an end of the test sheet on the side of application of gold colloid to obtain a detecting reagent for thyroglobulin.

### 〈5〉 Practice of detecting method based on use of detecting reagent for thyroglobulin (immunochromato method)

Thyroglobulin (Thyroglobulin, Human, produced by Chemicon International, Inc.) was diluted with Tris buffer described above to prepare a thyroglobulin solution adjusted to 1 µg/ml. The thyroglobulin solution and Tris buffer described above were used as specimens. The absorbing pad of the detecting reagent for thyroglobulin was directly immersed in the specimen. Sufficient absorption of the specimen was confirmed. After that, the detecting reagent for thyroglobulin was held horizontally and stationarily maintained. The specimen started to develop in accordance with the same phenomenon as that observed in paper chromatography, in accordance with which the galactose-deficient anti-thyroglobulin monoclonal antibody-immobilized gold colloid in a dry state also developed. When the development flow arrived at the portion of application of RCA 120, no coloration was observed for the specimen of Tris buffer, and the gold colloid merely made passage. On the contrary, in the case of the specimen of the thyroglobulin solution, red-purple coloration was observed. The operation consisted of one step of immersing the absorbing pad in the specimen, which was simple and convenient. The time required to obtain the decision was about 4 minutes. Thus we succeeded in detecting thyroglobulin conveniently and quickly.

As described above, the present invention provides the detecting reagent for thyroglobulin to be used for the immunochromato method based on the use of RCA 120.

### Industrial Applicability

The present invention provides the following industrially applicable effects.
(1) There is provided a detecting reagent used for a detecting method which makes it possible to detect a substance to be detected accurately, conveniently, and quickly.
(2) There is provided a detecting reagent for detecting an anti-galactose-deficient IgG antibody or a glycoprotein having a sugar chain containing galactose and/or β-N-acetylgalactosamine at its terminal in a human body fluid more quickly and conveniently as compared with the conventional quantitative method.
(3) There is provided a diagnostic reagent which allows accurate, convenient, and quick diagnosis of rheumatoid arthritis.

## Claims

1. A detecting reagent for detecting a substance to be detected having a sugar chain containing galactose and/or β-N-acetylgalactosamine at its terminal, the detecting reagent comprising a membrane on which a first reactive substance is immobilized and fine particles on which a second reactive substance is immobilized, wherein one of the reactive substances is Ricinus communis agglutinin I, and the other of the reactive substances is a reactive substance which specifically binds to the substance to be detected, and has no sugar chain containing galactose and β-N-acetylgalactosamine at its terminal.

2. The detecting reagent according to claim 1, wherein the substance to be detected is an anti-galactose-deficient IgG antibody, and the reactive substance which has no sugar chain containing galactose and β-N-acetylgalactosamine at its terminal is galactose-deficient IgG.

3. A detecting reagent for detecting an anti-galactose-deficient IgG antibody, the detecting reagent comprising a membrane and fine particles, wherein Ricinus communis agglutinin I is immobilized on one of the membrane and the fine particles, and galactose-deficient IgG is immobilized on the other.

4. The detecting reagent according to claim 3, wherein the galactose-deficient IgG is immobilized on the membrane, and Ricinus communis agglutinin I is immobilized on the fine particles.

5. The detecting reagent according to claim 1, wherein the substance to be detected is thyroglobulin, and the reactive substance which has no sugar chain containing galactose and β-N-acetylgalactosamine at its terminal is a galactose-deficient anti-thyroglobulin antibody.

6. A diagnostic agent for rheumatoid arthritis comprising the detecting reagent as defined in claim 3 or 4.

7. A method for detecting rheumatoid arthritis, comprising the steps of:
reacting a membrane on which one of galactose-deficient IgG and Ricinus communis agglutinin I is immobilized, colored fine particles on which the other is immobilized, and a biological sample collected from a test subject with each other; and
detecting the colored fine particles captured on the membrane through binding between an anti-galactose-deficient IgG antibody contained in the sample and the galactose-deficient IgG and binding between Ricinus communis agglutinin I and galactose contained in the anti-galactose-deficient IgG antibody.
